# EUROPÄISCHE PATENTSCHRIFT

(11) **EP 3 366 260 B1**
(45) Veröffentlichungstag und Bekanntmachung des Hinweises auf die Patenterteilung: **01.01.2020**
(21) Anmeldenummer: 18157873.3
(22) Anmeldetag: 21.02.2018
(51) Int. Cl.: A61C 13/00, A61C 13/09, A61C 13/083, A61K 6/02, A61C 5/77

(54) **VERFAHREN ZUM HERSTELLEN EINES ZAHNERSATZES**
METHOD FOR PRODUCING A DENTAL PROSTHETIC
PROCÉDÉ DE FABRICATION D'UNE PROTHÈSE DENTAIRE

(30) Priorität: 24.02.2017 AT 501522017
(43) Veröffentlichungstag der Anmeldung: 29.08.2018
(73) Patentinhaber: STEGER, Heinrich, 39031 Bruneck (IT)
(72) Erfinder: STEGER, Heinrich, 39031 Bruneck (IT)
(74) Vertreter: Torggler & Hofinger Patentanwälte

(56) Entgegenhaltungen:
- WO-A1-2007/028787
- WO-A1-2011/041194
- US-A- 4 104 798
- US-A- 5 314 334

## Beschreibung

Die Erfindung betrifft ein Verfahren zum Herstellen eines Zahnersatzes, insbesondere Krone, Brücke oder Steg, mit den Schritten Herstellen eines metallischen Gerüsts, Herstellen einer keramischen Verblendung, Aufbringen zumindest einer Fügeschicht auf das metallische Gerüst und/oder auf die keramische Verblendung und Aufbringen der keramischen Verblendung auf das metallische Gerüst, wobei die Fügeschicht zwischen metallischem Gerüst und keramischer Verblendung angeordnet ist. Zudem betrifft die Erfindung einen Zahnersatz hergestellt in einem solchen Verfahren.

Bei der Herstellung von Zahnersätzen in Form von Metall-Keramik-Kronen besteht das Grundgerüst aus einer metallischen Legierung, welches die Belastung auf den (darunter liegenden restlichen) Zahn überträgt und gleichzeitig für die Festigkeit und die Stabilität des Zahnersatzes sorgt. Dieses Gerüst aus Metall wird dann verblendet, wobei diese Verblendung mit einem keramischen Material erfolgt.

Bei einem bekannten Verfahren wird die Keramik als eine Paste auf das Metall händisch aufgetragen und dann in einem Keramikofen bei einer Temperatur von ca. 800° C gebrannt. Dies kann dann in mehreren Schritten erfolgen, um mehrere Schichten aufzubrennen. Bei einem solchen Herstellungsverfahren werden dann auch handwerkliche Geschicke vom durchführenden Zahntechniker erwartet, um einen ästhetisch zufriedenstellenden Zahnersatz zu erhalten.

Eine andere Möglichkeit besteht darin, dass das metallische Grundgerüst und die keramische Verblendung in einer CNC-gesteuerten Bearbeitungsmaschine hergestellt werden. In diesem Fall entfällt das manuelle Aufschichten, um eine erste Verblendung auf dem Metall zu erhalten. Speziell die keramische Verblendung kann dann noch mit der bekannten Methode der keramischen Schichttechnik verfeinert werden um so einen noch natürlicher wirkenden Zahnersatz zu erhalten. In einem nächsten Schritt wird dann diese so hergestellte Verblendung auf das metallische Grundgerüst zementiert. Dafür werden in der Regel selbst- oder lichthärtende Kompositkleber verwendet. Diese haben jedoch den Nachteil, dass, sobald die beiden Teile miteinander verklebt sind, keine zusätzliche Keramikschicht aufgebrannt werden kann, da der Kleber den Temperaturen bei einem Brennvorgang nicht standhält.

In der gattungsfremden DE 10 2005 023 106 A1 geht es um ein Verfahren zur Herstellung eines Zahnersatzteils. Dabei besteht das Zahnersatzteil aus einem mindestens eingliedrig ausgeführtem Gerüst und einem Verblendteil. Die Herstellung dieser Teile kann in CAD/CAM-Verfahren durchgeführt werden. Als Material für das erste Bauteil wird vorteilhafterweise Aluminiumoxid oder Zirkonoxid vorgesehen und für das zweite Bauteil eine Feldspatkeramik. Gattungsfremd ist diese Schrift deshalb, da nur in der Einleitung bei der Beschreibung des Stand der Technik beschrieben wird, dass als Kernmaterialien oft Metalle und als Verblendmaterialien dann Keramiken verwendet werden, jedoch ist die darin beschriebene Erfindung gerade nicht auf die Verwendung dieser Materialien ausgelegt. Das Befestigen der beiden Teile miteinander erfolgt dann durch Fügen, wobei ein Verbundmaterial verwendet wird. Als Verbundmaterial kann Glaslot, niedrigschmelzende Keramik oder organischer Kleber verwendet werden. Die zu verbindenden Bauteile sind bezüglich ihres Ausdehnungskoeffizienten vorteilhafterweise zueinander passend ausgewählt. Weiters wird auch angeführt, dass die beiden Bauteile über eine Wärmebehandlung zusammengefügt werden.

In der DE 197 14 759 B4 wird ein Zahnersatz in Form einer Zahnkrone beschrieben, welcher aus einem kappenartigen metallischen Innenteil und einem aufgesetzten gesinterten oxidischen Teil besteht. Der metallische Innenteil wird mit einem oxidischen Pulver beschichtet, wodurch eine Zwischenschicht gebildet wird. Im Speziellen wird durch ein thermisches Spritzen ein reines Aluminiumoxidpulver auf das Innenteil aufgetragen. Zum Auftragen der Aluminiumoxidschicht wird ein Plasmabrenner benötigt, was als aufwendiger Schritt für die Beschichtung anzusehen ist. Anschließend wird die gesondert hergestellte Zahnkeramik aufgesteckt und im Temperaturbereich von 650°C bis 850°C aufgesintert. Um die Farbgebung des Zahnersatzes zu beeinflussen wird unter anderem Zirkon als Oxid angeführt. Als Material für das Innenteil werden Titan oder Titanlegierungen vorgesehen. Wie genau die einzelnen Teile hergestellt werden wird in dieser Schrift nicht näher erläutert.

Die Aufgabe der vorliegenden Erfindung besteht darin, die genannten Nachteile zu vermeiden und ein gegenüber dem Stand der Technik verbessertes Verfahren bzw. einen verbesserten Zahnersatz zu schaffen.

Dies wird durch ein Verfahren mit den Merkmalen von Anspruch 1 erreicht. Demnach ist der Schritt gemeinsames Brennen des metallischen Gerüsts, der Fügeschicht und der keramischen Verblendung vorgesehen, wodurch die Fügeschicht das metallische Gerüst mit der keramischen Verblendung stoffschlüssig verbindet, wobei der Anteil an Titan oder einer Titanlegierung am metallischen Gerüst bei über 80 Gew.-% liegt, wobei die keramische Verblendung als Hauptbestandteil Zirconiumdioxid aufweist und der Zirconiumdioxid-Anteil bei mindestens 50 Gew.-% liegt, und wobei die Fügeschicht zu mehr als 60 Gew.-% aus Siliziumdioxid und zu weniger als 12 Gew.-% aus Aluminiumoxid besteht.

Zahnersatze mit anderen Zusammensetzungen gehen beispielsweise aus der US 5,314,334, der WO 2011/041194 A1, der US 4,104,798 und der WO 2007/028787 A1 hervor.

Um den Zahnersatz noch natürlicher aussehen zu lassen, sind die weiteren Schritte Aufbringen zumindest einer keramischen Verblendschicht auf die keramische Verblendung und gemeinsames Brennen des metallischen Gerüsts, der Fügeschicht, der keramischen Verblendung und der Verblendschicht vorgesehen. Die Verblendschicht kann dabei nur bereichsweise auf die keramische Verblendung aufgebracht werden. Bevorzugt wird die gesamte, nicht an die Fügeschicht angrenzende Oberfläche der keramischen Verblendung mit der Verblendschicht bedeckt.

Unter Verblendung werden nicht zwingend Elemente verstanden, welche einen metallischen Teil abdecken und somit "verblenden". Darunter können auch Elemente verstanden werden, welche einfach an ein metallisches Bauteil gefügt werden müssen. Vereinfacht kann somit gesagt werden, dass unter der Herstellung eines Zahnersatzes verstanden wird, dass ein metallisches Element (in diesem Fall als Gerüst ausgeführt) und ein keramisches Element (in diesem Fall als Verblendung ausgeführt) zusammengefügt werden.

Gemäß einem bevorzugten Ausführungsbeispiel ist vorgesehen, dass das metallische Gerüst in einer CNC-Bearbeitungsmaschine hergestellt, vorzugsweise gefräst, wird. Alternativ oder zusätzlich kann auch die keramische Verblendung in einer CNC-Bearbeitungsmaschine hergestellt, vorzugsweise gefräst, werden.

Weiters ist bevorzugt vorgesehen, dass die keramische Verblendung vor dem Aufbringen der Fügeschicht in einem Sinterofen dichtgesintert wird. Zusätzlich oder alternativ kann vorgesehen sein, dass das metallische Gerüst vor dem Aufbringen der Fügeschicht in einem Sinterofen dichtgesintert wird. Das Dichtsintern erfolgt jeweils bei einer für das jeweilige Material passenden Sintertemperatur.

Generell kann die Fügeschicht vor dem Zusammenfügen auf das metallische Gerüst oder auf die keramische Verblendung oder auf beide Teile aufgebracht werden.

Besonders bevorzugt ist vorgesehen, dass die Fügeschicht hauptsächlich aus, vorzugsweise silikatischer, Glaskeramik besteht. Eine derartige Fügeschicht hält die Einbrenntemperaturen des Keramikbrandes aus. Somit ist auch die Durchführung zumindest eines weiteren Brennvorgangs - vor allem nach dem Aufbringen der Verblendschicht - möglich, ohne dass das Risiko besteht, dass sich die Verblendung unerwünscht vom Gerüst löst.

Das metallische Gerüst kann auch nur einem einzigen Metall oder aus einer Metalllegierung besteht. Bevorzugt ist vorgesehen, dass das metallische Gerüst hauptsächlich aus Titan oder aus einer Titanlegierung besteht. Es können auch noch andere Metalle beigemengt sein. Im Speziellen ist der Anteil an Titan bzw. der Titanlegierung am metallischen Gerüst bei über 80 %, vorzugsweise bei über 88 %, Gewichtsprozent. Gemäß einem bevorzugten Ausführungsbeispiel wird als Material für das metallische Gerüst Titan 5 (ASTM-Standard) mit folgenden Eigenschaften verwendet:

| | |
|---|---|
| Dichte: | 4,43 g/cm³ |
| Vickers Härte: | 341 HV |
| Wärmeausdehnungskoeffizient (25 - 500° C) | 9,7-1·10⁶⁻·K⁻¹ |
| Thermische Leitfähigkeit: | 6,7 W/(mK) |
| Chemische Zusammensetzung (%): | Titan (ca. 90 %), Aluminium (ca. 6 %), Vanadium (ca. 4 %), Eisen und Sauerstoff (jeweils geringfügige Anteile), |
| Zugfestigkeit: | 860 MPa |
| Streckgrenze: | 790 MPa |
| Ausdehnung: | 15 % |
| E-Modul: | 114 GPa |
| Schermodul: | 44 GPa |
| Poissonzahl: | 0,342 |

Unter Keramik für die keramische Verblendung ist ein anorganischer nichtmetallischer Werkstoff zu verstehen. Generell können alle Keramiken oder Keramikmischungen verwendet werden, die für dentale Anwendungen geeignet sind. Bevorzugt ist vorgesehen, dass die keramische Verblendung als Hauptbestandteil Zirconiumdioxid aufweist. Dies bedeutet, dass der Zircioniumdioxid-Anteil bei mindestens 50 Gewichtsprozent liegt. Bevorzugt ist vorgesehen, dass der Anteil an Zirconiumdioxid bei über 85 Gewichtsprozent liegt.

Gemäß einem ersten Ausführungsbeispiel wird für die keramische Verblendung ein Material mit folgenden Eigenschaften verwendet:

| | |
|---|---|
| Zirconiumdioxid (ZrO₂) | Hauptbestandteil (über 50 Gew.-%) |
| Yttriumoxid (Y₂O₃) | Anteil von < 12 Gewichtsprozent |
| Aluminiumoxid (Al₂O₃) | Anteil von < 1 Gewichtsprozent |
| Siliciumdioxid (SiO₂) | Anteil max. 0,02 Gewichtsprozent |
| Eisen(III)-oxid (Fe₂O₃) | Anteil max. 0,02 Gewichtsprozent |
| Dichte (g/cm³): | ∼ 6,0 g/cm³ |
| Biegefestigkeit: | 670 MPa |
| Vickershärte (HV10) | 1250 HV10 |
| Weibull-Modul | ∼ 5 |
| Wärmeausdehnungskoeffizient: | ∼ 10,0·10⁻⁶·K⁻¹ |

Zusätzlich kann in diesem Material auch noch Hafniumoxid enthalten sein.

Gemäß einem zweiten Ausführungsbeispiel kann für die keramische Verblendung ein Material mit folgenden Eigenschaften verwendet werden:

| | |
|---|---|
| Zirconiumdioxid (ZrO₂) | Hauptbestandteil |
| Yttriumoxid (Y₂O₃) | Anteil von 4 - 6 Gewichtsprozent |
| Aluminiumoxid (Al₂O₃) | Anteil von < 1 Gewichtsprozent |
| Siliciumdioxid (SiO₂) | Anteil max. 0,02 Gewichtsprozent |
| Eisen(III)-oxid (Fe₂O₃) | Anteil max. 0,01 Gewichtsprozent |
| Natriumoxid (Na₂O) | Anteil max. 0,04 Gewichtsprozent |
| Dichte: | ∼ 6,0 g/cm³ |
| Biegefestigkeit (bei R.T.) | 1000 - 1200 MPa |
| Vickershärte: | 1250 HV10 |
| Weibull-Modul: | ∼ 15 |
| WAK (Wärmeausdehnungskoeffizient) | ∼ 10,0·10⁻⁶·K⁻¹ |

Auch in diesem Material kann zusätzlich noch Hafniumoxid enthalten sein.

Um Sprünge im Zahnersatz oder das Brechen des Zahnersatzes durch das (mehrmalige) Brennen zu vermeiden, ist bevorzugt vorgesehen, dass sich der Wärmeausdehnungskoeffizient der Fügeschicht um maximal 2,0·10⁻⁶·K⁻¹, vorzugsweise um maximal 1,0·10⁻⁶·K⁻¹, von den Wärmeausdehnungskoeffizienten sowohl des metallischen Gerüsts als auch der keramischen Verblendung unterscheidet. Durch diese relativ geringen Unterschiede bei der Wärmeausdehnung ist auch das Risiko von Sprüngen niedriger als bei großen Unterschieden.

Eine besonders gute Resistenz gegen Sprünge besteht dann, wenn der Wärmeausdehnungskoeffizient der Fügeschicht zwischen dem Wärmeausdehnungskoeffizient des metallischen Gerüsts und dem Wärmeausdehnungskoeffizient der keramischen Verblendung liegt. Natürlich kann der Wärmeausdehnungskoeffizient der Fügeschicht aber auch - vorzugsweise geringfügig - oberhalb oder unterhalb der Wärmeausdehnungskoeffizienten des metallischen Gerüsts und der keramischen Verblendung liegen. Im Speziellen ist bevorzugt vorgesehen, dass der Wärmeausdehnungskoeffizient der Fügeschicht um maximal 1,7·10⁻⁶·K⁻¹ niedriger ist als der Wärmeausdehnungskoeffizient der keramischen Verblendung. Zudem kann bevorzugt vorgesehen sein, dass der Wärmeausdehnungskoeffizient der Fügeschicht um maximal 1,5·10⁻⁶·K⁻¹ Wärmeausdehnungskoeffizient des metallischen Gerüsts abweicht.

Konkret kann vorgesehen sein, dass das metallische Gerüst einen Wärmeausdehnungskoeffizient von ca. 9,7·10⁻⁶·K⁻¹ aufweist. Die keramische Verblendung sollte einen Wärmeausdehnungskoeffizient von 10,0·10⁻⁶·K⁻¹ aufweisen. Der Wärmeausdehnungskoeffizient der Fügeschicht liegt bevorzugt in einem Bereich zwischen 8,0 und 10,5·10⁻⁶·K⁻¹. Bevorzugt weist die Fügeschicht einen Wärmeausdehnungskoeffizient von 10,0·10⁻⁶·K⁻¹ auf. Es versteht sich, dass diese Werte auch von den hier angegebenen Bereichen abweichen können. Es sollte jedoch sichergestellt werden, dass die Unterschiede der Koeffizienten zueinander zulässige Differenzen einhalten.

Für die Wärmeausdehnungskoeffizienten gelten die Angaben der Hersteller der einzelnen Komponenten auf den zugehörigen Datenblättern. Falls keine solchen Angaben vorhanden sind, kann auch eine Messung des Wärmeausdehnungskoeffizienten erfolgen. Für die Messung des Wärmeausdehnungskoeffizienten kann generell ein Dilatometer verwendet werden. Beispielhaft sei hierzu der Dilatometer mit dem Markennamen "DIL 402 C" der Firma NETZSCH-Gerätebau GmbH angeführt. Für keramische Werkstoffe im Bereich der Zahnheilkunde kann die Wärmeausdehnungskoeffizientmessung auf Basis der DIN EN ISO 6872 in der Fassung von 2015 erfolgen. In dieser Norm wird unter Punkt 7.4 die Messung des linearen thermischen Ausdehnungskoeffizienten beschrieben. Für die Messung bei metallischen Werkstoffen im Bereich der Zahnheilkunde sollte die DIN EN ISO 22674 in der Fassung von 2016 herangezogen werden. Die Testmethode zur Messung der linearen thermischen Ausdehnung ist darin unter Punkt 8.13 beschrieben.

Wenn die Fügeschicht aus einer silikatischen Glaskeramik besteht ist bevorzugt vorgesehen, dass diese zu mehr als 60 Gewichtsprozent, vorzugsweise zu 60 bis 68 Gewichtsprozent, aus Siliziumdioxid und zu weniger als 12 Gewichtsprozent, vorzugsweise zwischen 5 bis 12 Gewichtsprozent, aus Aluminiumoxid besteht. Zusätzlich kann auch noch vorgesehen sein, dass diese Fügeschicht einen Anteil an Kaliumoxid zwischen 4 und 9 Gewichtsprozent und einen Anteil an Natriumoxid zwischen 5,5 und 12 Gewichtsprozent aufweist. Die Fügeschicht dient ja vor allem dazu, dass eine gute stoffschlüssige Verbindung mit der keramischen Verblendung sichergestellt wird. Zudem sollte die Fügeschicht auch geeignete optische Eigenschaften aufweisen. Um sicherzustellen, dass das darunter liegende, metallische Gerüst nicht zu deutlich zu erkennen ist, ist in einer möglichen Ausführungsform vorgesehen, dass die Fügeschicht über zumindest eine Abdeckschicht mit dem metallischen Gerüst verbunden wird. Somit ist die Fügeschicht nur indirekt über diese Abdeckschicht mit dem metallischen Gerüst stoffschlüssig verbunden. Dasselbe gilt auch, wenn auf der keramischen Verblendung zumindest eine Abdeckschicht aufgebracht wird. Dadurch ist die Fügeschicht indirekt über die Abdeckschicht mit der keramischen Verblendung stoffschlüssig verbunden. Diese Abdeckschicht sollte relativ opak sein. Konkret kann auch für diese Abdeckschicht eine silikatische Glaskeramik verwendet werden. Es kann auch vorgesehen sein, dass mehrere dieser Abdeckschichten aufgetragen werden, bevor die keramische Verblendung aufgebrannt wird. Diese einzelnen Schichten können sich dann auch in ihrer Zusammensetzung voneinander unterscheiden.

Schutz wird auch begehrt für einen Zahnersatz, insbesondere Krone, Brücke oder Steg, hergestellt in einem erfindungsgemäßen Verfahren, mit einem metallischen Gerüst, einer keramischen Verblendung und einer das Gerüst und die Verblendung verbindenden Fügeschicht. Hierzu sei auch erwähnt, dass das erfindungsgemäße Verfahren bei allen zahntechnischen Arbeiten funktioniert. Somit soll sich die Erfindung nicht nur auf Kronen, Brücken und Stege beschränken. Beispielsweise können auch zirkuläre Arbeiten oder auch Teleskoparbeiten über dieses Verfahren hergestellt werden, um nur einige Beispiele zu nennen. Bevorzugt ist auch auf die keramische Verblendung zumindest eine Verblendschicht zumindest bereichsweise aufgebracht. Sämtliche hierin beschriebenen Ausführungsbeispiele und bevorzugte Varianten gelten - sofern logisch sinnvoll - auch für den Zahnersatz.

Weitere Einzelheiten und Vorteile der vorliegenden Erfindung werden anhand der Figurenbeschreibung unter Bezugnahme auf die in den Zeichnungen dargestellten Ausführungsbeispiele im Folgenden näher erläutert. Darin zeigen:
- Fig. 1: schematisch den Aufbau eines Zahnersatzes,
- Fig. 2: schematisch den einzelnen Schritte des Herstellungsverfahrens dieses Zahnersatzes,
- Fig. 3: schematisch ein metallisches Gerüst in Form von vier Zahnstümpfen mit einer aufgebrachten keramischen Verblendung,
- Fig. 4: eine perspektivische Ansicht eines Abutments und
- Fig. 5: Frontansichten von Gerüsten in Form von Titanbasen.

Der innerste Teil des Zahnersatzes Z ist das metallische Gerüst 1. Auf dieses metallische Gerüst 1 ist die Fügeschicht 2 aufgeschichtet. Darauf folgt die keramische Verblendung 3. Auf diesem ist wiederum eine (oder mehrere) Verblendschicht 4 aufgebracht.

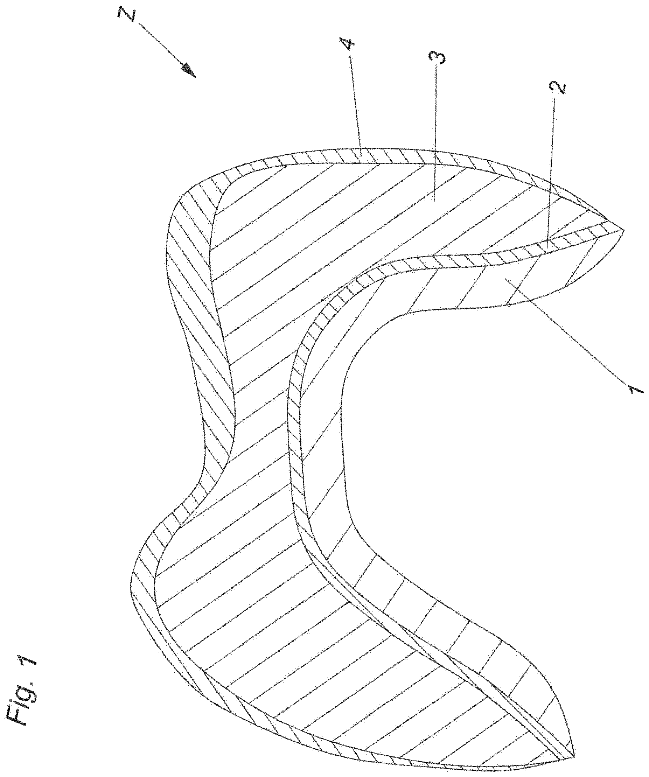

Bei der in Fig. 1 dargestellten ersten Variante wird das metallische Gerüst 1, welches hauptsächlich aus Titan oder einer Titanlegierung besteht, mit einem zweiten Teil (keramische Verblendung 3) aus Zirconiumdioxid verblendet. Beide Teile werden mit Hilfe einer CAD-CAM-Vorrichtung modelliert und auch gefertigt. Das Verbinden der beiden Teile, auch als Fügeprozess bezeichnet, erfolgt über eine Fügemasse (Fügeschicht 2). Als Fügemassen können grundsätzlich Materialien verwendet werden, welche einen Wärmeausdehnungskoeffizient haben, welcher ähnlich dem Wärmeausdehnungskoeffizienten der beiden zu fügenden Bauteile ist.

Im konkreten Fall werden keramische Kronen, Brücken oder ähnliche zahntechnische Elemente aus Zirconiumdioxid verwendet, welche eine thermische Ausdehnung von ca. 10,0·10⁻⁶·K⁻¹ aufweisen. Die verwendeten metallischen Gerüste 1 aus Titan oder Titanlegierungen weisen eine thermische Ausdehnung von ca. 9,7·10⁻⁶·K⁻¹ auf. Als Material für die Fügeschicht 2 können organische oder anorganische Mittel verwendet werden. So können Materialien wie Glaslot, Keramiken oder auch organische Kleber zum Einsatz kommen. Bevorzugt wird aber eine silicatische Glaskeramik mit einer thermischen Ausdehnung von ca. 10,0·10⁻⁶·K⁻¹ verwendet. Die thermischen Ausdehnungskoeffizienten können jedoch je nach genauer Zusammensetzung schwanken. Prinzipiell kann der Fügevorgang jedoch auch mit anderen Materialien durchgeführt werden, welche in etwa einen gewünschten Ausdehnungskoeffizienten haben. Die silicatische Glaskeramik weist Bestandteile von SiO₂, Al₂O₃, K₂O, Na₂O, CaO und B₂O₃ auf. Ganz bevorzugt basiert die Fügekeramik auf Leucit-freien silicatischen Sintergläsern.

Die Zusammensetzung möglicher Pulver kann aus der der folgenden Tabelle entnommen werden (Massenanteile in Gewichtsprozent).

| Komponenten | Material A | Material B | Material C |
|---|---|---|---|
| SiO₂ | 63 - 68 | 60 - 65 | 58 - 62 |
| Al₂O₃ | 5 - 8 | 8 - 12 | 7 - 11 |
| K₂O | 5.5 - 9 | 4 - 6 | 5 - 8 |
| Na₂O | 5.5 - 9 | 8 - 12 | 8 - 12 |
| Li₂O | 1 - 3 | - | 1 - 3 |
| CaO | 1 - 3 | 0.1 - 2 | 1 - 3 |
| SrO | 1.5 - 4 | - | - |
| BaO | - | 4 - 6 | - |
| B₂O₃ | 0 - 3 | 4 - 6 | 3 - 5 |
| P₂O₅ | < 0.2 | - | 4 - 6 |
| CeO₂ | 0 - 2 | - | 0.5 - 2.5 |
| ZnO | 2 - 5 | - | 0.1 - 2 |
| SnO₂ | - | - | 0.1 - 2 |
| MgO | - | 0.1 - 2 | - |
| ZrO₂ | 0 - 2 | - | - |
| TiO₂ | 1 - 3 | - | - |
| F | 0-2 | 0.1 - 2 | - |
| WAK-Wert | 10,0·10⁻⁶·K⁻¹ | 8,5·10⁻⁶·K⁻¹ | 9,0·10⁻⁶·K⁻¹ |

Je nach Farbe des Produktes werden dann noch Pigmente beigemischt. Dies kann einen Anteil von bis zu 30% ausmachen. Bei Tests mit 100% Material A (welches transparenter ist als das Material C in gebrannten Zustand) als Fügeschicht 2, wurde eine sehr gute stoffschlüssige Verbindung zwischen dem metallischen Gerüst 1 und der keramischen Verblendung 3 erreicht.

Alternativ wurde eine gute stoffschlüssige Verbindung auch dadurch erreicht, dass zunächst eine erste Schicht des Material C als Abdeckschicht aufgetragen und gebrannt wurde, um das metallische Gerüst 1 zu verdecken, wonach das Material A mit der keramischen Verblendung 3 aufgebracht und gebrannt wurde.

In einem weiteren Versuch wurde das Material A mit Material C vermengt (Aufteilung 50:50) und dann direkt aufgetragen und die Krone (keramische Verblendung 3) darüber gesetzt und alles gebrannt. In diesem Fall konnte auch eine zufriedenstellende Verbindung erzielt werden.

Das als keramisches Material verwendete Zirconiumdioxid wird umgangssprachlich auch als Zirkonoxid oder Zirkon bezeichnet und ist ein in der Dentalbranche häufig verwendetes Material, da es eine hervorragende Biokompatibilität aufweist und auch den ästhetischen Ansprüchen an einen hochwertigen Zahnersatz Z genügt. Zudem weist es auch die nötigen mechanischen Eigenschaften beispielsweise hinsichtlich Bruchfestigkeit oder auch Verschleißfestigkeit auf. An sich können jedoch auch andere Keramiken (z. B. technische Keramiken oder Glaskeramiken) zum Einsatz kommen, welche die nötigen Eigenschaften aufweisen. Zwei bevorzugt Varianten wurden bereits weiter oben angeführt, welche einen Wärmeausdehnungskoeffizienten von ca. 10,0·10⁻⁶·K⁻¹ aufweisen. Das in den Versuchen verwendete Titan Grad 5 hat einen Ausdehnungskoeffizienten von ca. 9,7·10⁻⁶·K⁻¹.

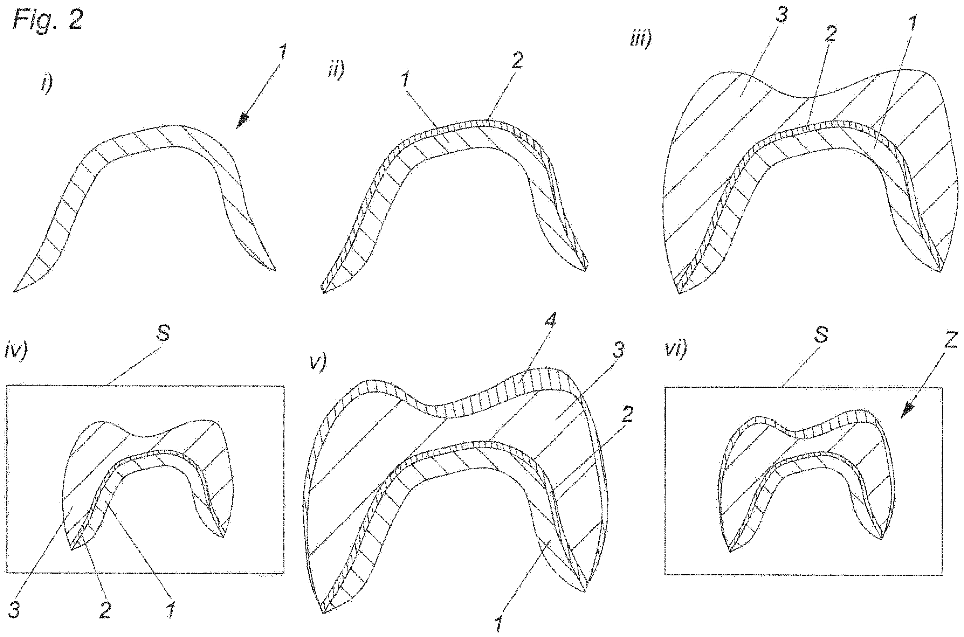

Mit Bezug auf Fig. 2 werden im Folgenden die Schritte für das Verfahren zum Herstellen eines Zahnersatzes näher erläutert.

In einem ersten Schritt (nicht dargestellt) werden die Daten vom Mund des Patienten erfasst (z. B. die Oberfläche des zumindest einen Zahnstumpfes, die Lage der Implantate usw.), um dann über eine geeignete Software die nötige Modellierung des Gerüstes und der Verblendung durchführen zu können. Dies kann mittels eines Computers in einem Erfassungs- und Modellierungsmodus durchgeführt werden. Die entsprechende Software ist dafür in einem Speicher des Computers hinterlegt. Das metallische Gerüst wird dann beispielsweise an den aufgenommenen Zahnstumpf angepasst. Auch die keramische Verblendung wird entsprechend modelliert. Zwischen metallischem Gerüst und keramischer Verblendung wird bevorzugt auch schon ein Spalt vorgesehen, welcher zwischen 0,01 mm und 0,5 mm liegen kann, bevorzugt aber zwischen 0,02 mm und 0,2 mm und ganz bevorzugt bei ca. 0,05 mm liegt.

Nachdem die beiden Teile metallisches Gerüst 1 aus Titan und keramische Verblendung 3 aus Zirkon, zum Beispiel in einer CAD/CAM-Bearbeitungsvorrichtung, hergestellt worden sind (siehe Schritt i in Fig. 2, welcher nur das metallische Gerüst 1 zeigt), wird das metallische Gerüst 1 mit einer ersten Schicht der Glaskeramik (Fügeschicht 2) bedeckt. Dies ist in Schritt ii von Fig. 2 gezeigt. Alternativ zur in Fig. 2 dargestellten Variante kann die Fügeschicht 2 auch auf die keramische Verblendung 3 aufgebracht werden. Es kann auch auf beide Teile (1 und 3) eine dann jeweils dünnere Fügeschicht 2 aufgetragen werden.

Bevor dieser Schritt ii durchgeführt wird, ist bevorzugt vorgesehen, dass die Oberfläche des metallischen Gerüsts 1 sandgestrahlt wird. Hierzu können die unterschiedlichsten Granulate verwendet werden. Eine Möglichkeit ist Aluminiumoxid mit einer Korngröße von ca. 50-200 µm bei ca. 2-3 bar. Dies hat den Vorteil, dass die Oberfläche aufgeraut wird, wodurch ein besserer Haftverbund erzeugt werden kann. Dies kann sowohl beim metallischen Gerüst 1 als auch bei der keramischen Verblendung 2 durchgeführt werden.

Vor dem Auftragen der Fügeschicht 2 wird das Pulver mit einer Flüssigkeit zu einer aufstreichbaren Paste angerührt. Diese wird dann mit einem Pinsel auf das metallische Gerüst 1 mit einer Stärke von ca. 0,5 mm aufgetragen. Bevorzugt entspricht die aufgetragene Schichtdicke dem Spalt, welcher zwischen den beiden Teilen bei der Modellierung vorgesehen worden ist. Dieser Vorgang kann auch automatisiert durchgeführt werden, wie beispielsweise über einen Sprühmechanismus, über eine sonstige Aufbringvorrichtung oder über eine geeignete Methode (beispielsweise Tauchen). In einem solchen Fall muss die Konsistenz an den verwendeten Prozess angepasst werden. Bei dieser Fügeschicht 2 ist darauf zu achten, dass die gesamte Oberfläche des metallischen Gerüstes 1 damit bedeckt wird, damit kein darunterliegender Teil mehr sichtbar ist. Diese Fügeschicht 2 wird von der Zusammensetzung dann bevorzugt auch so gewählt, dass es sich um eine opake und nicht transluzente Masse handelt. Dies hat den Vorteil, dass die graue Struktur des metallischen Gerüstes 1 verdeckt wird und somit nicht durch die keramische Verblendung 3 durchschimmert. Dadurch können optisch bessere Ergebnisse bei der Herstellung des Zahnersatzes Z erzielt werden.

Nachdem die Glaskeramik (Fügeschicht 2) aufgetragen wurde, wird dann die vorher bereits gefertigte keramische Verblendung 3 darüber gesteckt (siehe Schritt iii in Fig. 2). Dabei kann es dazu kommen, dass zu viel aufgetragenes Material aus dem Fügespalt herausgedrückt wird. Dieses wird dann entfernt. Hierbei ist dann darauf zu achten, dass die keramische Verblendung 3 an die dafür vorgesehen Position auf dem metallischen Gerüst 1 aufgedrückt wird.

Anschließend wird dieser "Dreierverbund" gebrannt bzw. gesintert. Dazu wird der Verbund erst an der Luft für ca. 5 min. getrocknet. Dann wird der Verbund in ca. 5 min. in den Brennofen S eingefahren (siehe Schritt iv in Fig. 2), welcher eine Bereitschaftstemperatur von ca. 400° C hat und dann mit einer Steigerung von ca. 55 K/min in einem Vakuum auf 800° C erhitzt. In der Regel folgt dann noch eine Haltezeit von einer Minute. Anschließend wird dann der Brennofen S langsam, auch in Stufenschritten, geöffnet und die gefügten Teile langsam herausgefahren. Dadurch wird erreicht, dass ein kontrolliertes Abkühlen erfolgt. Dieser Schritt bedarf an die zehn Minuten. Der hier beschriebene Vorgang stellt eine bevorzugte Variante dar und kann auch mit anderen Zeit- und Temperaturparametern durchgeführt werden.

Der Vorteil einer solchen Behandlung besteht darin, dass dieser gefügte Verbund dann noch mit weiterer Keramik (Verblendschicht 4; siehe Schritt v in Fig. 2) beschichtet und verfeinert werden kann. Daraufhin können sogenannte Glasurbrände im Brennofen S (siehe Schritt vi in Fig. 2) durchgeführt werden, ohne den durch die Fügeschicht 2 gegebenen Haftverbund zwischen dem metallischen Gerüst 1 und der keramischen Verblendung 3 zu gefährden.

Nach dem Stand der Technik wurden bisher die Verblendungen mit der Keramikpaste beschichtet, und diese immer wieder gebrannt, um die Festigkeit der Keramik zu erhalten. Das wurde so lange durchgeführt, bis das gewünschte Ergebnis erzielt wird. Diese mit Keramik verblendete Verblendung wurde dann über Zement, beispielsweise einen Kompositkleber, mit dem Metallgerüst verbunden. Wurde dieser Zahnersatz dann im Mund des Patienten eingesetzt und festgestellt, dass die Farbgebung nicht ganz zu den bisherigen Zähnen passt, dann musste man den Verbund wieder lösen, um weitere Anpassungen bei der Keramik bezüglich Farbe machen zu können, da bei einem erneuten Brennen im Ofen der Kleber verbrennen würde.

Bei Verwendung der Fügeschicht 2, vorzugsweise in Form einer silicatischen Glaskeramik, entfallen diese Probleme und man kann beliebig oft noch Anpassungen vornehmen. Die keramischen Schichtmassen der Fügeschicht 2 können dann mit beliebiger Pigmentierung, vorteilhafterweise nach der VITA-Farbscala A1-D4, ausgewählt werden.

Unter Umständen kann auch die keramische Verblendung 3 selber schon eingefärbt sein. Dies wird mit sogenannten Liquids durchgeführt, mit welchen die keramische Verblendung 3 vor dem Dichtsintern eingefärbt wird. Das Dichtsintern der Verblendung 3 im Falle von Zirconiumdioxid erfolgt bei ca. 1.400° bis 1.500° C. Dieser Temperaturbereich kann jedoch variieren. Dieses Dichtsintern der keramischen Verblendung erfolgt in einem Sinterofen (nicht gezeigt) vor dem Fügen. Es kann auch vorgesehen sein, dass für die keramische Verblendung 3 mehrschichtige Blöcke verwendet werden, welche bereits unterschiedliche Farbschichten aufweisen und nicht zwingend eingefärbt werden müssen. Zur Herstellung der keramischen Verblendung 3 werden in der Regel gepresste Rohlinge verwendet, welche nach der Bearbeitung dann, wie oben beschrieben, dichtgesintert werden. Es kann jedoch auch vorgesehen sein, dass keramische Rohlinge verwendet werden, welche bereits ihre Enddichte besitzen und nicht mehr dichtgesintert werden müssen. Diese werden dann nur noch oberflächlich, vorzugsweise durch Schleifen, bearbeitet.

Bei einer zweiten Variante aufbauend auf der in Fig. 2 dargestellten ersten Variante kann auch vorgesehen sein, dass eine erste Schicht der silicatischen Glaskeramik (Fügeschicht 2) auf das metallische Gerüst 1 aufgetragen wird. Anschließend wird dann das metallische Gerüst 1 mit dieser ersten aufgetragenen Schicht gebrannt, ohne aufgesetzte keramische Verblendung 3.

Anschließend wird dann das metallische Gerüst 1 mitsamt der Fügeschicht 2 mit einem Scanner erfasst um die neuen geometrischen Daten der Oberfläche zu erhalten. Aufbauend auf diesen Daten kann dann wieder die keramische Verblendung 3 modelliert werden. Hier kann dann wieder ein Spalt vorgesehen werden zwischen den beiden Teilen.

Theoretisch ist es auch möglich, dass der Scanvorgang entfällt und die zuvor modellierte Verblendung 3 auf die bereits gebrannte Fügeschicht 2 aufgesetzt wird und dann dieser Verbund nochmal gebrannt wird. Jedoch auch in diesem Fall kann die Verblendung 3 mithilfe eines Scans angepasst werden.

Erfolgt dies nicht, kann im nächsten Schritt erneut eine Fügeschicht 2 der silicatischen Glaskeramik aufgetragen werden. In diesem Fall kann sich die Fügemasse aber auch von der ersten verwendeten Masse unterscheiden. Es ist nicht zwingend vorgesehen, dass dieselbe Masse verwendet werden muss.

Theoretisch kann jetzt diese aufgetragene Schicht wieder gebrannt werden, über einen Scanner dann erfasst werden und die Modellierung wieder durchgeführt werden. Im Normalfall wird jedoch, bevor diese zweite aufgetragene Schicht gebrannt wird, die Verblendung 3 aufgesetzt und dann wieder der komplette Verbund gebrannt. Dies wird nach der vorhin beschriebenen Prozedur durchgeführt.

Bei einer dritten Variante kann auch vorgesehen sein, dass das metallische Gerüst 1, bevor es mit der Fügeschicht 2 beschichtet wird, schon mit einem Scanner digital erfasst wird. In manchen Fällen kann es vorkommen, dass am metallischen Gerüst 1 noch manuell Anpassungen vorgenommen werden müssen, beispielsweise parallelisieren der Wände. Diese Änderungen müssen dann natürlich wieder digitalisiert werden, um den Anschluss der keramischen Verblendung 3 dementsprechend modellieren zu können.

Nachdem dieser Schritt erfolgt ist, kann dann nach der ersten oder auch zweiten Variante weiter verfahren werden.

Wird davon ausgegangen, dass das Titan des metallischen Gerüsts 1 einen geringeren thermischen Ausdehnungskoeffizienten aufweist als die keramische Verblendung 3, dann kann beim Auftragen von mehreren Fügeschichten 2 vorgesehen sein, dass sich die einzelnen Schichten auch in ihrem thermischen Ausdehnungskoeffizienten unterscheiden und das so, dass sich dieser langsam vom Titan an die Verblendung anpasst. Beispielsweise hat das Titan eine Wärmeausdehnungskoeffizient von 9,0·10⁻⁶·K⁻¹ und das Zirkon von 10,5·10⁻⁶·K⁻¹. So könnten zwei Schichten vorgesehen werden, wobei die erste Schicht am Titan eine thermische Ausdehnung von 9,5·10⁻⁶·K⁻¹ hat und die zweite Schicht von 10,0·10⁻⁶·K⁻¹ Somit kann ein fließender Übergang erzeugt werden. Hier kann auch mit mehreren Schichten gearbeitet werden.

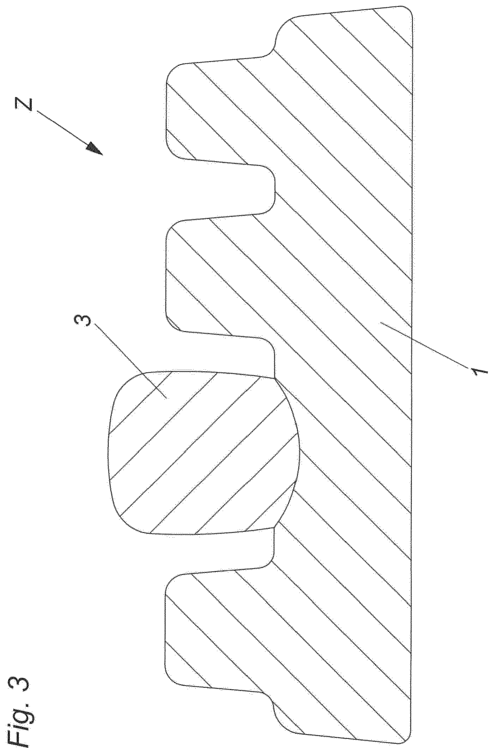

Wie bereits erwähnt, kann die vorliegende Erfindung nicht nur für Einzelarbeiten, sondern auch für größere Arbeiten verwendet werden. So kann gemäß Fig. 3 beispielsweise vorgesehen sein, dass das metallische Gerüst 1 eine Struktur umfasst, welche vier einzelne Zahnstümpfe aufweist, über die dann die einzelnen keramischen Verblendungen 3 gefügt werden können. Diese Verblendungen 3 können einzeln vorliegen oder selber als ein zusammenhängender Teil ausgeführt sein. Dieser Zahnersatz Z kann nach dem Fügen dann wieder mit einer Verblendschicht 4 verblendet werden. Auch der untere, noch frei liegende Titanteil, welcher auch im Mund des Patienten sichtbar wäre, kann dann mit einer keramischen Verblendschicht 4 verblendet werden, um beispielsweise das Zahnfleisch zu imitieren. Dafür werden dann auch keramische Massen mit einer ästhetisch passenden Pigmentierung verwendet.

Alternativ kann auch sein Steg gefertigt werden, welcher komplett in der Verblendung 3 eingesetzt werden kann und dann nichts mehr vom metallischen Gerüst 1 zu sehen ist, oder nur sehr kleine Bereiche, welche wieder mit Keramik verblendet werden können.

Generell ist es auch möglich, dass die metallischen Gerüste 1 und auch die keramischen Verblendungen 3 auf andere Art und Weise hergestellt werden, wie beispielsweise über diverse in der Dentalbranche bekannte Gussverfahren.

Auch können andere dentale Legierungen verwendet werden, welche einen Wärmeausdehnungskoeffizienten haben, welcher mit der Verblendung und dem Fügemittel ähnlich ist. Als Metall für dentale Anwendungen wäre zum Beispiel eine Chrom-Kobalt-Legierung (CrCo) geeignet.

Bei einer bevorzugten Ausführungsvariante wird das metallische Gerüst 1 aus einem massiven Block herausgerarbeitet/gefräst. Alternativ kann auch ein Sintermetallblock verwendet werden. In diesem Fall muss dann das metallische Gerüst 1, bevor die Fügeschicht 2 aufgetragen wird, dichtgesintert werden.

Auch ist es möglich, dass die keramische Verblendung 3, bevor sie auf das metallische Gerüst 1 gefügt wird, mit einer Verblendschicht 4 manuell beschichtet wird und dies vorher gebrannt, bevor diese komplette Verblendung (3 und 4) dann auf das metallische Gerüst 1 gefügt wird. So würde die optische Anpassung an die restlichen Schritte schon in einem ersten Prozessschritt erfolgen, also noch vor dem Fügevorgang. Anschließend, nach dem Fügevorgang, können dann immer noch Verfeinerungen vorgenommen werden.

Der Brenn- bzw. Sinterprozess kann dann noch nach Bedarf an die genau verwendeten Materialien angepasst werden. So kann beispielsweise eine langsamere Aufheizrate verwendet werden, um weniger Spannungen im Material zu erzeugen oder auch um die Start- und Endtemperatur anzupassen.

Bei den verwendeten Massen für die Fügeschicht 2 ist es auch möglich, dass unterschiedliche Pulver vermengt werden. So kann ein Pulver beispielsweise darauf abzielen, die Gerüststruktur zu verbergen und ist dementsprechend opak (Abdeckschicht). Dieses wird dann mit einem Pulver vermengt, welches transluzent ist, um dadurch ein weniger opakes Pulver zu generieren. Dieses Pulver wird dann wieder mit einer Flüssigkeit zu einer auftragbaren Paste vermengt und kann dann aufgeschichtet und nach den oben schon beschriebenen Verfahren verarbeitet werden.

Eine Möglichkeit besteht natürlich auch immer, dass eines der beiden zu fügenden Teile, oder auch beide, manuell hergestellt werden und dann gefügt werden. Wird eines manuell hergestellt, dann kann dieses über einen Scanner erfasst werden und das zweite Teil wird dann dementsprechend angepasst/modelliert und hergestellt.

Weiters kann es auch sein, dass die Verblendung 3 selber mehrschichtig aufgebaut ist und die einzelnen Schichten miteinander verbunden/gefügt werden. Diese einzelnen Schichten können dann wiederum über eine CAD/CAM-Vorrichtung automatisiert erzeugt werden.

Zur Herstellung können neben materialabtragenden Verfahren auch materialaufbauende, additive Verfahren verwendet werden. Auch Fräsen, Schleifen, generative Fertigungsverfahren usw. sind möglich. Generell können alle Fertigungsverfahren angewendet werden, welche aus der DIN 8580 hervorgehen und über welche sich die einzelnen Bestandteile herstellen lassen.

Im Folgenden wird noch als abschließender (nicht dargestellter) Schritt die Befestigung im Mund des Patienten beschrieben.

Ein wie in Fig. 1 dargestellter Zahnersatz Z kann dann, wenn er fertig ist, auf einen Zahnstumpf im Mund des Patienten zementiert werden. Hierzu kann der natürliche Zahn im Mund des Patienten so bearbeitet worden sein, dass der Zahnersatz Z dann aufgesteckt werden kann. Wie eingangs schon beschreiben, muss zum Erstellen des Zahnersatzes Z in einem ersten Schritt die Situation im Mund des Patienten digital erfasst werden. Hierzu können die unterschiedlichsten Methoden zum Einsatz kommen. Zum einen ist die Erfassung über einen Intraoralscanner denkbar. Es können aber auch Abdrücke genommen werden, welche dann wiederum digital erfasst werden. Hier sind dem Zahntechniker die unterschiedlichsten Verfahren bekannt.

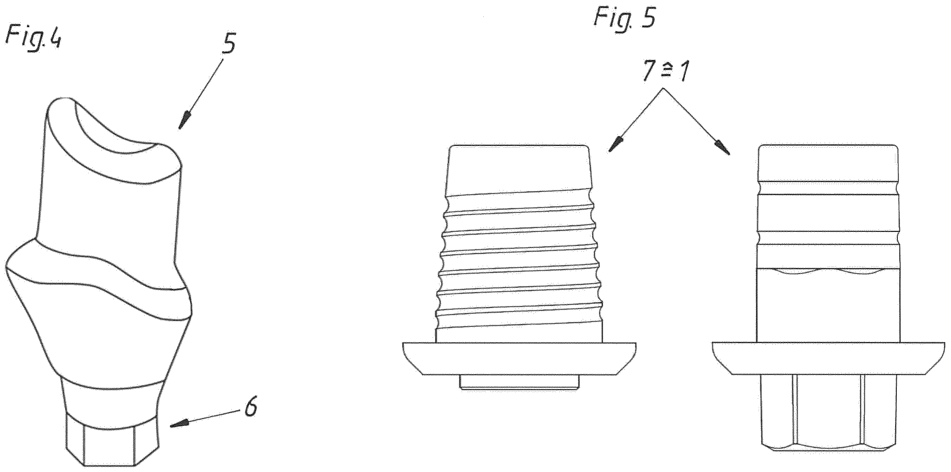

Es kann auch sein, dass der Patient ein Implantat im Kieferknochen hat und darauf ein Abutment 5 fixiert ist. Ein solches Abutment 5 ist in Fig. 4 dargestellt. Dieses Abutment 5 hat die Form eines Stumpfes, auf welchen dann wieder der Zahnersatz Z (hier nicht dargestellt) aufgesetzt/zementiert werden kann. Das Abutment 5 wird dann über seinen Verbindungsbereich 6 mit dem Implantat im Kieferknochen verdrehsicher verbunden und über eine Schraube am Implantat fixiert. Es besteht jedoch auch die Möglichkeit, dass der Zahnersatz Z über eine Schraubverbindung mit dem Implantat verbunden wird. Es ist auch möglich, dass als metallisches Gerüst 1 eine im Handel übliche Titanbase 7 verwendet wird, auf welche dann die Verblendung 3 gefügt wird. Mögliche Ausgestaltungen von Titanbasen 7 sind in Fig. 5 dargestellt. Diese Titanbasen 7 weisen in der Regel einen Schraubensitz auf und können dann mit dem Implantat über eine Schraube verbunden werden. Alternativ kann auch das metallische Gerüst 1 einen Anschluss aufweisen, welcher mit einer Titanbase 7 kompatibel ist. Bei solchen Varianten kann es dann nötig sein, dass die keramische Verblendung 3 über eine Durchgangsbohrung verfügt, über welche dann die Schraube eingeführt werden kann, um den Zahnersatz Z im Mund des Patienten zu fixieren.

Prinzipiell gibt es hier noch die unterschiedlichsten Möglichkeiten, einen Zahnersatz Z im Mund eines Patienten zu fixieren. Der Umstand, dass diese hier nicht alle beschrieben sind, bedeutet nicht, dass die Erfindung in einer solchen Kombination nicht funktioniert, sondern vielmehr, dass diese Praktiken einem Zahntechniker bekannt sind und nicht alle aufgelistet werden müssen.

### Bezugszeichenliste:

- Z: Zahnersatz
- S: Brennofen
- 1: metallisches Gerüst
- 2: Fügeschicht
- 3: keramische Verblendung
- 4: Verblendschicht
- 5: Abutment
- 6: Verbindungsbereich
- 7: Titanbase

## Patentansprüche

1. Verfahren zum Herstellen eines Zahnersatzes (Z), insbesondere Krone, Brücke oder Steg, mit den Schritten
- Herstellen eines metallischen Gerüsts (1), wobei der Anteil an Titan oder einer Titanlegierung am metallischen Gerüst (1) bei über 80 Gew.-% liegt,
- Herstellen einer keramischen Verblendung (3), wobei die keramische Verblendung (3) als Hauptbestandteil Zirconiumdioxid aufweist, wobei der Zirconiumdioxid-Anteil bei mindestens 50 Gew.-% liegt,
- Aufbringen zumindest einer Fügeschicht (2) auf das metallische Gerüst (1) und/oder auf die keramische Verblendung (3), wobei die Fügeschicht (2) zu mehr als 60 Gew.-% aus Siliziumdioxid und zu weniger als 12 Gew.-% aus Aluminiumoxid besteht,
- Aufbringen der keramischen Verblendung (3) auf das metallische Gerüst (1), wobei die Fügeschicht (2) zwischen metallischem Gerüst (1) und keramischer Verblendung (3) angeordnet ist, und
- gemeinsames Brennen des metallischen Gerüsts (1), der Fügeschicht (2) und der keramischen Verblendung (3), wodurch die Fügeschicht (2) das metallische Gerüst (1) mit der keramischen Verblendung (3) stoffschlüssig verbindet.

2. Verfahren nach Anspruch 1, mit den anschließenden Schritten:
- zumindest bereichsweises Aufbringen zumindest einer keramischen Verblendschicht (4) auf die keramische Verblendung (3) und
- gemeinsames Brennen des metallischen Gerüsts (1), der Fügeschicht (2), der keramischen Verblendung (3) und der Verblendschicht (4).

3. Verfahren nach wenigstens einem der vorhergehenden Ansprüche, wobei das metallische Gerüst (1) und/oder die keramische Verblendung (3) in einer CNC-Bearbeitungsmaschine hergestellt werden/wird.

4. Verfahren nach wenigstens einem der vorhergehenden Ansprüche, wobei die keramische Verblendung (3) und/oder das metallische Gerüst (1) vor dem Aufbringen der Fügeschicht (2) jeweils in einem Sinterofen (S) dichtgesintert werden/wird.

5. Verfahren nach wenigstens einem der vorhergehenden Ansprüche, wobei das metallische Gerüst (1) zu zumindest 88 Gew.-% aus Titan oder aus einer Titanlegierung besteht.

6. Verfahren nach wenigstens einem der vorhergehenden Ansprüche, wobei der Zircioniumdioxid-Anteil der keramischen Verblendung bei über 85 Gew.-% liegt.

7. Verfahren nach wenigstens einem der vorhergehenden Ansprüche, wobei sich der Wärmeausdehnungskoeffizient der Fügeschicht (2) um maximal 2,0·10⁻⁶·K⁻¹, vorzugsweise um maximal 1,0·10⁻⁶·K⁻¹, von den Wärmeausdehnungskoeffizienten sowohl des metallischen Gerüsts (1) als auch der keramischen Verblendung (3) unterscheidet.

8. Verfahren nach wenigstens einem der vorhergehenden Ansprüche, wobei der Wärmeausdehnungskoeffizient der Fügeschicht (2) zwischen dem Wärmeausdehnungskoeffizient des metallischen Gerüsts (1) und dem Wärmeausdehnungskoeffizient der keramischen Verblendung (3) liegt.

9. Verfahren nach wenigstens einem der vorhergehenden Ansprüche, wobei der Wärmeausdehnungskoeffizient der Fügeschicht (2) um maximal 1,7·10⁻⁶·K⁻¹ niedriger ist als der Wärmeausdehnungskoeffizient der keramischen Verblendung (3).

10. Verfahren nach wenigstens einem der vorhergehenden Ansprüche, wobei der Wärmeausdehnungskoeffizient der Fügeschicht (2) um maximal 1,5·10⁻⁶·K⁻¹ vom Wärmeausdehnungskoeffizient des metallischen Gerüsts (1) abweicht.

11. Verfahren nach wenigstens einem der vorhergehenden Ansprüche, wobei die Fügeschicht (2) zu 60 bis 68 Gewichtsprozent aus Siliziumdioxid und zwischen 5 bis 12 Gew.-% aus Aluminiumoxid besteht.

12. Verfahren nach Anspruch 11, wobei die Fügeschicht (2) einen Anteil an Kaliumoxid zwischen 4 und 9 Gewichtsprozent und einen Anteil an Natriumoxid zwischen 5,5 und 12 Gew.-% aufweist.

13. Verfahren nach wenigstens einem der vorhergehenden Ansprüche, wobei die Fügeschicht (2) über eine Abdeckschicht mit dem metallischen Gerüst (1) und/oder mit der keramischen Verblendung (3) verbunden wird.

14. Zahnersatz (Z), insbesondere Krone, Brücke oder Steg, hergestellt in einem Verfahren nach einem der Ansprüche 1 bis 13, mit
- einem metallischen Gerüst (1), wobei der Anteil an Titan oder einer Titanlegierung am metallischen Gerüst (1) bei über 80 Gew.-% liegt,
- einer keramischen Verblendung (3), wobei die keramische Verblendung (3) als Hauptbestandteil Zirconiumdioxid aufweist, wobei der Zirconiumdioxid-Anteil bei mindestens 50 Gew.-% liegt, und
- einer das Gerüst (1) und die Verblendung (3) verbindenden Fügeschicht (2), wobei die Fügeschicht (2) zu mehr als 60 Gew.-% aus Siliziumdioxid und zu weniger als 12 Gew.-% aus Aluminiumoxid besteht,.

15. Zahnersatz nach Anspruch 14, wobei auf die keramische Verblendung (3) zumindest eine Verblendschicht (4) zumindest bereichsweise aufgebracht ist.

## Claims

1. A method for producing a denture (Z), in particular a crown, a bridge or a bar, with the steps
- producing a metallic framework (1), wherein the proportion of titanium or a titanium alloy in the metallic framework (1) is above 80 % by weight,
- producing a ceramic veneer (3), wherein the ceramic veneer (3) comprises zirconium dioxide as a main component, wherein the proportion of zirconium dioxide is at least 50 % by weight,
- applying at least one joining layer (2) onto the metallic framework (1) and/or onto the ceramic veneer (3), wherein the joining layer (2) contains a proportion of silicon dioxide of more than 60 % by weight and a proportion of aluminium oxide of less than 12 % by weight,
- applying the ceramic veneer (3) onto the metallic framework (1), wherein the joining layer (2) is arranged between the metallic framework (1) and the ceramic veneer (3), and
- joint burning of the metallic framework (1), the joining layer (2) and the ceramic veneer (3), whereby the joining layer (2) firmly bonds the metallic framework (1) with the ceramic veneer (3).

2. The method according to claim 1, with the subsequent steps:
- at least partially applying of at least one ceramic veneer layer (4) onto the ceramic veneer (3) and
- joint burning of the metallic framework (1), the joining layer (2), the ceramic veneer (3) and the veneer layer (4).

3. The method according to at least one of the previous claims, wherein the metallic framework (1) and/or the ceramic veneer (3) are/is produced in a CNC processing machine.

4. The method according to at least one of the previous claims, wherein the ceramic veneer (3) and/or the metallic framework (1) each are/is densely sintered in a sintering oven (S) before applying the joining layer (2).

5. The method according to at least one of the previous claims, wherein the metallic framework (1) consists of at least 88 % by weight of titanium or a titanium alloy.

6. The method according to at least one of the previous claims, wherein the proportion of zirconium dioxide in the ceramic veneer (3) is above 85 % by weight.

7. The method according to at least one of the previous claims, wherein the coefficient of thermal expansion of the joining layer (2) is different from the coefficient of thermal expansion of the metallic framework (1) and of the ceramic veneer (3) by a maximum of 2.0·10⁻⁶·K⁻¹ preferably by a maximum of 1.0·10⁻⁶·K⁻¹.

8. The method according to at least one of the previous claims, wherein the coefficient of thermal expansion of the joining layer (2) is between the coefficient of thermal expansion of the metallic framework (1) and of the coefficient of thermal expansion of the ceramic veneer (3).

9. The method according to at least one of the previous claims, wherein the coefficient of thermal expansion of the joining layer (2) is lower than the coefficient of thermal expansion of the ceramic veneer (3) by a maximum of 1.7·10⁻⁶·K⁻¹.

10. The method according to at least one of the previous claims, wherein the coefficient of thermal expansion of the joining layer (2) differs from the coefficient of thermal expansion of the metallic framework (1) by a maximum of 1.5·10⁻⁶·K⁻¹.

11. The method according to at least one of the previous claims, wherein the joining layer (2) consists by a proportion of 60 to 68 percent by weight of silicone dioxide and by a proportion of 5 to 12 percent by weight of aluminium oxide.

12. The method according to claim 11, wherein the joining layer (2) comprises a proportion of potassium oxide between 4 and 9 percent by weight and a proportion of sodium oxide between 5.5 and 12 percent by weight.

13. The method according to at least one of the previous claims, wherein the joining layer (2) is connected to the metallic framework (1) and/or to the ceramic veneer (3) by a covering layer.

14. A denture (Z), in particular a crown, a bridge or a bar, produced in a method according to one of the claims 1 to 13, comprising
- a metallic framework (1), wherein the proportion of titanium or a titanium alloy in the metallic framework (1) is above 80 % by weight,
- a ceramic veneer (3), wherein the ceramic veneer (3) comprises zirconium dioxide as a main component, wherein the proportion of zirconium dioxide is at least 50 % by weight, and
- a joining layer (2) which connects the framework (1) and the veneer (3), wherein the joining layer (2) contains a proportion of silicon dioxide of more than 60 % by weight and a proportion of aluminium oxide of less than 12 % by weight.

15. The denture according to claim 14, wherein a veneer layer (4) is at least partially applied onto the ceramic veneer (3).

## Revendications

1. Procédé de fabrication d'une prothèse dentaire (Z), en particulier d'une couronne, d'un bridge ou d'une barre, comprenant les étapes consistant à:
- fabriquer une architecture métallique (1), la proportion de titane ou d'un alliage de titane dans l'architecture métallique (1) étant à plus de 80 % en poids,
- fabriquer un revêtement en céramique (3), le revêtement en céramique (3) présentant du dioxyde de zirconium comme ingrédient principal, la proportion de dioxyde de zirconium étant à moins de 50 % en poids,
- déposer au moins une couche de jonction (2) sur l'architecture métallique (1) et/ou sur le revêtement en céramique (3), la couche de jonction (2) se composant à plus de 60 % en poids de dioxyde de silicium et à moins de 12 % en poids d'oxyde d'aluminium,
- déposer le revêtement en céramique (3) sur l'architecture métallique (1), la couche de jonction (2) étant disposée entre l'architecture métallique (1) et le revêtement en céramique (3), et
- calciner conjointement l'architecture métallique (1), la couche de jonction (2) et le revêtement en céramique (3), moyennant quoi la couche de jonction (2) relie par liaison de matière l'architecture métallique (1) au revêtement en céramique (3).

2. Procédé selon la revendication 1, comprenant les étapes consécutives suivantes consistant à :
- déposer au moins par zones au moins une couche de revêtement en céramique (4) sur le revêtement en céramique (3) et
- calciner conjointement l'architecture métallique (1), la couche de jonction (2), le revêtement en céramique (3) et la couche de revêtement (4).

3. Procédé selon au moins l'une des revendications précédentes, dans lequel l'architecture métallique (1) et/ou le revêtement en céramique (3) est/sont fabriqués dans une machine d'usinage CNC.

4. Procédé selon au moins l'une des revendications précédentes, dans lequel le revêtement en céramique (3) et/ou l'architecture métallique (1) est/sont densément frittés respectivement dans un four de frittage (S) avant le dépôt de la couche de jonction (2).

5. Procédé selon au moins l'une des revendications précédentes, dans lequel l'architecture métallique (1) se compose à au moins 88 % en poids de titane ou d'un alliage de titane.

6. Procédé selon au moins l'une des revendications précédentes, dans lequel la proportion de dioxyde de zirconium du revêtement en céramique est à plus de 85 % en poids.

7. Procédé selon au moins l'une des revendications précédentes, dans lequel le coefficient de dilatation thermique de la couche de jonction (2) se distingue d'au maximum 2,0·10⁻⁶·K⁻¹, de préférence d'au maximum 1,0·10⁻⁶·K⁻¹, des coefficients de dilatation thermique aussi bien de l'architecture métallique (1) que du revêtement en céramique (3).

8. Procédé selon au moins l'une des revendications précédentes, dans lequel le coefficient de dilatation thermique de la couche de jonction (2) se situe entre le coefficient de dilatation thermique de l'architecture métallique (1) et le coefficient de dilatation thermique du revêtement en céramique (3).

9. Procédé selon au moins l'une des revendications précédentes, dans lequel le coefficient de dilatation thermique de la couche de jonction (2) est inférieur d'au maximum 1,7·10⁻⁶·K⁻¹ au coefficient de dilatation thermique du revêtement en céramique (3).

10. Procédé selon au moins l'une des revendications précédentes, dans lequel le coefficient de dilatation thermique de la couche de jonction (2) est inférieur d'au maximum 1,5·10⁻⁶·K⁻¹ au coefficient de dilatation thermique de l'architecture métallique (1).

11. Procédé selon au moins l'une des revendications précédentes, dans lequel la couche de jonction (2) se compose de 60 à 68 % en poids de dioxyde de silicium et de 5 à 12 % en poids d'oxyde d'aluminium.

12. Procédé selon la revendication 11, dans lequel la couche de jonction (2) présente une proportion d'oxyde de potassium entre 4 et 9 % en poids et une proportion d'oxyde de sodium entre 5,5 et 12 % en poids.

13. Procédé selon au moins l'une des revendications précédentes, dans lequel la couche de jonction (2) est reliée à l'architecture métallique (1) et/ou au revêtement en céramique (3) par le biais d'une couche de recouvrement.

14. Prothèse dentaire (Z), en particulier couronne, bridge ou barre, fabriquée au cours d'un procédé selon l'une des revendications 1 à 13, comprenant
- une architecture métallique (1), la proportion de titane ou d'alliage de titane dans l'architecture métallique (1) étant à plus de 80 % en poids,
- un revêtement en céramique (3), le revêtement en céramique (3) comprenant du dioxyde de zirconium comme ingrédient principal, la proportion de dioxyde de zirconium étant à au moins 50 % en poids, et
- une couche de jonction (2) reliant l'architecture (1) et le revêtement (3), la couche de jonction (2) se composant à plus de 60 % en poids de dioxyde de silicium et à moins de 12 % en poids d'oxyde d'aluminium.

15. Prothèse dentaire selon la revendication 14, dans laquelle au moins une couche de revêtement (4) est déposée au moins par zones sur le revêtement en céramique (3).
